# EUROPÄISCHE PATENTSCHRIFT

(11) **EP 0 754 676 B1**
(45) Veröffentlichungstag und Bekanntmachung des Hinweises auf die Patenterteilung: **03.11.1999**
(21) Anmeldenummer: 96111236.4
(22) Anmeldetag: 12.07.1996
(51) Int. Cl.: C07C 209/60

(54) **Verfahren zur Herstellung von Aminen aus Olefinen an Zeolithen des Typs PSH-3, MCM-22, SSZ-25 oder deren Gemischen**
Process for the preparation of amines from olefines using PSH-3, MCM-22, SSZ-25 zeolithes or mixtures thereof
Procédé pour la préparation d'amines à partir d'oléfines utilisant des zéolithes des types PSH-3, MCM-22, SSZ-25 ou leurs mélanges

(30) Priorität: 20.07.1995 DE 19526502
(43) Veröffentlichungstag der Anmeldung: 22.01.1997
(73) Patentinhaber: BASF AKTIENGESELLSCHAFT, 67056 Ludwigshafen (DE)
(72) Erfinder: Eller, Karsten, Dr., 67059 Ludwigshafen (DE); Müller, Ulrich, Dr., 67434 Neustadt (DE); Kummer, Rudolf, Dr., 67227 Frankenthal (DE); Stops, Peter, 67122 Altrip (DE)

(56) Entgegenhaltungen:
- EP-A- 0 431 451
- EP-A- 0 587 424
- US-A- 5 149 894
- US-A- 5 264 635
- DATABASE WPI Section Ch, Week 9431 Derwent Publications Ltd., London, GB; Class A60, AN 94-249692 XP002029455 & CA 2 092 964 A (TEXACO CHEM CO) , 4.Juni 1994

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drücken in Gegenwart von Zeolithen des Typs PSH-3, MCM-22, SSZ-25 oder deren Gemischen.

Eine Übersicht über die Methoden zur Aminierung von Olefinen wird in "Functionalisation of Alkenes: Catalytic Amination of Monoolefins", J.J. Brunet et al. J.Mol.Catal., 49 (1989), Seiten 235 bis 259 gegeben.

Grundsätzlich gibt es zwei Katalysemechanismen. Das Olefin wird über ein Metallkomplex koordiniert. Diese aktivierte Spezies kann von dem nucleophilen Amin angegriffen werden und ein höher aminiertes Produkt bilden. Das Amin kann an Säurezentren oder an Metallzentren (via Metallamide) chemisorbiert werden und so aktiviert mit dem Olefin umgesetzt werden.

Gut geeignete Katalysatoren sind Zeolithe. Sie zeichnen sich durch eine hohe Anzahl an katalytisch aktiven Zentren aus, kombiniert mit einer großen Oberfläche. Die beschriebenen Zeolithe unterscheiden sich im Typ und in der Nachbehandlung (z.B. thermische Behandlung, Dealuminierung, Säurebehandlung, Metallioneneintausch, etc.). Beispiele hierfür finden sich in US-A-4 375 002, US-A-4 536 602, EP-A-305 564, EP-A-101 921, DE-A-42 06 992.

Aus EP-A-133 938, EP-A-431 451 und EP-A-132 736 sind Verfahren bekannt, bei denen Bor-, Gallium-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Å definiert sind, eingesetzt werden. Bevorzugt werden metall- oder halogenmodifizierte Beta-Zeolithe eingesetzt.

EP-A-587 424 betrifft ein Verfahren zur Herstellung von Aminen, insbesondere tert.-Butylamin, durch Umsetzung von Ammoniak und einfach oder mehrfach ungesättigten Olefinen mit 2 bis 10 C-Atomen in Gegenwart eines dealuminierten Y-Zeolithen. Die maximale Ausbeute an tert.-Butylamin beträgt 17,1%.

EP-A-431 451 betrifft ein Verfahren zur Herstellung von Aminen aus Olefinen und Ammoniak oder primären oder sekundären Aminen bei Temperaturen zwischen 60 und 400°C und einem Druck zwischen 40 und 700 bar in Gegenwart eines Galliumsilikat-Zeolithen des Pentasil-Typs. Die maximale Ausbeute an tert.-Butylamin beträgt 15,2%.

Alle Verfahren zur Synthese von Aminen aus Olefinen an diesen Katalysatoren zeichnen sich durch eine geringe Amin-Ausbeute oder geringe Raum-Zeit-Ausbeute aus, oder führen zu einer raschen Desaktivierung der Katalysatoren.

Der vorliegenden Erfindung lag daher die Aufgabe zugrunde, diesen Nachteilen abzuhelfen.

Demgemäß wurde ein neues und verbessertes Verfahren zur Herstellung von Aminen der allgemeinen Formel I 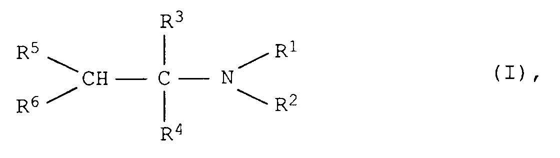 in der
- R¹,R²,R³,R⁴,R⁵,R⁶: Wasserstoff, C₁- bis C₂₀-Alkyl, C₂- bis C₁₂-Alkenyl, C₂- bis C₈-Alkinyl, C₃- bis C₁₂-Cycloalkyl, C₄- bis C₁₂-Alkyl-cycloalkyl, C₄- bis C₁₂-Cycloalkyl-alkyl, Aryl, C₇- bis C₁₆-Alkylaryl oder C₇- bis C₁₆-Aralkyl,
- R¹ und R²: gemeinsam eine gesättigte oder ungesättigte C₃-bis C₉-Alkylendikette und
- R³ und R⁵: gemeinsam eine C₂- bis C₁₂-Alkylendikette
bedeuten, durch Umsetzung von Olefinen der allgemeinen Formel II 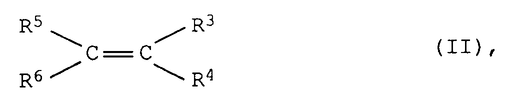 in der R³, R⁴, R⁵ und R⁶ die oben genannten Bedeutungen haben, mit Ammoniak oder primären oder sekundären Aminen der allgemeinen Formel III 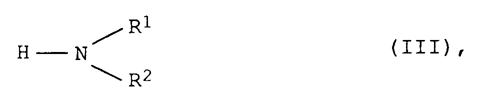 in der R¹ und R² die oben genannten Bedeutungen haben, bei Temperaturen von 200 bis 350°C und Drücken von 100 bis 300 bar in Gegenwart eines Heterogenkatalysators gefunden, welches dadurch gekennzeichnet ist, daß man als Heterogenkatalysator Zeolithe des Typs PSH-3, MCM-22, SSZ-25 oder deren Gemische einsetzt.

Das erfindungsgemäße Verfahren kann wie folgt durchgeführt werden:

Das Olefin II und Ammoniak oder das primäre oder sekundären Amin III kann bei Temperaturen von 200 bis 350°C, bevorzugt 220 bis 330°C, besonders bevorzugt 230 bis 320°C und Drücken von 100 bis 300 bar, bevorzugt 120 bis 300 bar, besonders bevorzugt 140 bis 290 bar in Gegenwart von Zeolithen des Typs PSH-3, MCM-22, SSZ-25 oder deren Gemischen als Katalysator z.B. in einem Druck-Reaktor umsetzt werden, und bevorzugt das erhaltene Amin abgetrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung des Katalysators zurückgedrängt worden.

Das erfindungsgemäße Verfahren zeichnet sich dadurch aus, daß bereits mit niedrigem Ammoniak- bzw. Amin- Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Als Katalysatoren für die Aminierung von Olefinen eignen sich Zeolithe des Typs PSH-3, MCM-22, SSZ-25 oder deren Gemische, bevorzugt MCM-22 Zeolithe, die beispielsweise aus Stud. Surf. Sci. Catal., 84, 331 bis 338 (1994) bekannt sind.

Aus der US-A-4,954,325 ist ein Zeolith mit der Bezeichnung MCM-22, dessen Porenverteilung zwischen der Porenverteilung eines ZSM-12 und der Porenverteilung eines Zeolith BETA liegt, bekannt. Aus der US-A-4,439,409 ist ein solcher MCM-22 Zeolith unter dem Namen PSH-3 und aus der EP-A-231 019 unter dem Namen SSZ-25 mit sehr ähnlichem Röntgendiffraktogramm bekannt.

Die erfindungsgemäßen Zeolithe MCM-22, PSH-3 und SSZ-25 können als solche verformt werden, oder aber auch mit einem Bindemittel im Verhältnis 98:2 bis 40:60 Gew.-% zu Strängen oder Tabletten. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Preßlinge zweckmäßig bei 110°C/16 h getrocknet und bei 200 bis 500°C/2 bis 16 h calciniert, wobei die Calcinierung auch direkt im Aminierungsreaktor erfolgen kann.

Zur Erhöhung der Selektivität, der Standzeit und der Anzahl der möglichen Regenerierungen kann man verschiedene Modifizierungen an den erfindungsgemäßen Zeolithkatalysatoren MCM-22, PSH-3 und SSZ-25 vornehmen.

Eine Modifizierung der Katalysatoren besteht darin, daß man die unverformten oder die verformten Zeolithe mit Alkalimetallen wie Na und K, Erdalkalimetallen wie Ca, Mg, Erdmetallen wie Tl, Übergangsmetallen wie z.B. Ti, Zr, Mn, Fe, Mo, Cu, Zn, Cr, Edelmetallen und/oder seltenen Erdmetallen wie z.B. La, Ce oder Y ionenaustauschen bzw. dotieren kann.

Eine vorteilhafte Ausführungsform besteht darin, daß man die verformten erfindungsgemäßen Zeolithe MCM-22, PSH-3 und SSZ-25 in einem Strömungsrohr vorlegt und bei 20 bis 100°C z.B. ein Halogenid, ein Acetat, ein Oxalat, ein Citrat oder ein Nitrat der oben beschriebenen Metalle in gelöster Form darüberleitet. Ein derartiger Ionenaustausch kann z.B. an der Wasserstoff-, Ammonium- und Alkaliform der erfindungsgemäßen Zeolithe MCM-22, PSH-3 und SSZ-25 vorgenommen werden.

Eine weitere Möglichkeit der Metallaufbringung auf die erfindungsgemäßen Zeolithe MCM-22, PSH-3 und SSZ-25 besteht darin, daß man das Material z.B. mit einem Halogenid, einem Acetat, einem Oxalat, einem Citrat, einem Nitrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten Zeolithen des Typs MCM-22, PSH-3 und SSZ-25 kann eine Nachbehandlung mit Wasserstoff und/oder mit Wasserdampf günstig sein.

Eine weitere Möglichkeit der Modifizierung besteht darin, daß man die erfindungsgemäßen Zeolithe MCM-22, PSH-3 und SSZ-25 - verformt oder unverformt - einer Behandlung mit Säuren, wie Salzsäure (HCl), Flußsäure (HF), Schwefelsäure (H₂SO₄), Phosphorsäure (H₃PO₄) oder Oxalsäure (HO₂C-CO₂H) unterwirft.

Eine besondere Ausführungsform besteht darin, daß man die erfindungsgemäßen Zeolithe MCM-22, PSH-3 und SSZ-25 vor der Verformung mit einer der genannten Säuren 0,001 n bis 2 n, bevorzugt 0,05 bis 0,5 n 1 bis 100 Stunden unter Rückfluß behandelt. Nach Abfiltrieren und Auswaschen wird in der Regel bei 100 bis 160°C getrocknet und bei 200 bis 600°C calciniert. Eine weitere besondere Ausführungsform liegt in einer Säure-Behandlung der erfindungsgemäßen Zeolithe MCM-22, PSH-3 und SSZ-25 nach ihrer Verformung mit Bindemittel. Hierbei wird der erfindungsgemäße Zeolith in der Regel 1 bis 3 Stunden zwischen 60 und 80°C mit einer 3 bis 25%igen, insbesondere mit einer 12 bis 20%igen Säure behandelt, anschließend ausgewaschen, bei 100 bis 160°C getrocknet und bei 200 bis 600°C calciniert.

Eine andere Möglichkeit der Modifizierung ist gegeben durch einen Austausch mit Ammonsalzen, z.B. mit NH₄Cl oder mit Mono-, Di- oder Polyaminen. Hierbei wird der mit Bindemittel verformte Zeolith in der Regel bei 60 bis 80°C mit 10 bis 25%iger, bevorzugt 20%iger NH₄Cl-Lösung 2 h kontinuierlich in gewichtsmäßiger Zeolith/Ammonchlorid-Lösung von 1:15 ausgetauscht und danach bei 100 bis 120°C getrocknet.

Eine weitere Modifikation, die an den erfindungsgemäßen Zeolithen vorgenommen werden kann, ist eine Dealuminierung, bei der ein Teil der Aluminiumatome durch Silicium ersetzt wird oder die Zeolithe durch beispielsweise hydrothermale Behandlung in ihrem Aluminiumgehalt abgereichert werden. An eine hydrothermale Dealuminierung schließt sich vorteilhafterweise eine Extraktion mit Säuren oder Komplexbildnern an, um gebildetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen von Y-Zeolithen finden sich in Corma et al., Stud. Surf. Sci. Catal. 37 (1987) Seiten 495 bis 503.

Die Katalysatoren kann man als Stränge mit Durchmessern von z.B. 1 bis 4 mm oder als Tabletten mit z.B. 3 bis 5 mm Durchmesser für die Aminierung der Olefine einsetzen.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
R¹,R²,R³,R⁴,R⁵,R⁶
   - Wasserstoff,
   - C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
   - C₂- bis C₁₂-Alkenyl, bevorzugt C₂- bis C₈-Alkenyl wie Vinyl und Allyl,
   - C₂- bis C₈-Alkinyl, insbesondere C₂H und Propargyl,
   - C₃- bis C₁₂-Cycloalkyl, bevorzugt C₅- bis C₈-Cycloalkyl wie Cyclopentyl, Cyclohexyl, Cycloheptyl und Cyclooctyl,
   - C₄- bis C₁₂-Alkyl-cycloalkyl, bevorzugt C₅- bis C₁₀-Alkyl-cycloalkyl,
   - C₄- bis C₁₂-Cycloalkyl-alkyl, bevorzugt C₅- bis C₁₀-Cycloalkyl-alkyl,
   - Aryl wie Phenyl, 1-Naphthyl und 2-Naphthyl, bevorzugt Phenyl,
   - C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
   - C₇- bis C₁₆-Aralkyl, bevorzugt C₇- bis C₁₂-Phenalkyl wie Phenylmethyl, 1-Phenylethyl, 2-Phenylethyl,
R¹ und R²
   - gemeinsam eine gesättigte oder ungesättigte C₃- bis C₉-Alkylendikette, bevorzugt -(CH₂)₄-, -(CH₂)₅-, -(CH₂)₇- und -CH=CH-CH=CH-,
R³ und R⁵
   - gemeinsam eine C₂- bis C₁₂-Alkylendikette, bevorzugt eine C₃- bis C₈-Alkylendikette, besonders bevorzugt -(CH₂)₃-, -(CH₂)₄-, -(CH₂)₅-, -(CH₂)₆- und -(CH₂)₇-, insbesondere -(CH₂)₃- und -(CH₂)₄-.

### Beispiele

### Katalysatorsynthese

30g MCM-22 wurden mit 20g Boehmit und 1g Ameisensäure versetzt, im Kneter kompaktiert und unter Wasserzusatz (52 ml) 45 Minuten verknetet. In einer Strangpresse wurden mit einem Preßdruck von 40 bar 2mm Stränge erzeugt, 16 h bei 120°C getrocknet und anschließend 16 h bei 500°C calciniert.

### Aminierungsbeispiele

Die Versuche wurden in einem Rohrreaktor (6 mm Innendurchmesser) unter isothermen Bedingungen bei 260°C bis 300°C und einem Druck von 280 bar mit einem Gemisch aus Isobuten und Ammoniak im molaren Verhältnis von 1:1,5 durchgeführt. Die Reaktionsprodukte wurden im Gaschromatographen analysiert.

Die Ergebnisse sind in Tabelle 1 zusammengestellt.

**Tabelle 1**

| tert.-Butylamin (NH₃: C₄H₈ = 1,5) | | | | | |
|---|---|---|---|---|---|
| Druck | Temperatur | tert.-Butylamin-Ausbeute [Gew.-%] | | | Litergewicht |
| [bar] | [°C] | WHSV 0,7 [g/g·h] | WHSV 1,5 [g/g·h] | WHSV 3 [g/g·h] | [kg/l] |
| 280 | 260 | 18,81 | | | 0.43 |
| 280 | 270 | 20,50 | 17,34 | 13,26 | 0.43 |
| 280 | 280 | 17,89 | 16,99 | 14,60 | 0.43 |
| 280 | 300 | | | 11,89 | 0.43 |

## Patentansprüche

1. Verfahren zur Herstellung von Aminen der allgemeinen Formel I 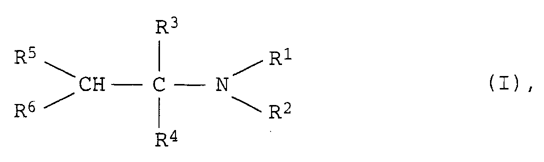 in der
R¹,R²,R³,R⁴,R⁵,R⁶ Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
R¹ und R² gemeinsam eine gesättigte oder ungesättigte C₃- bis C₉-Alkylendikette und
R³ und R⁵ C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 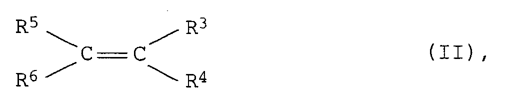 in der R³, R⁴, R⁵ und R⁶ die oben genannten Bedeutungen haben, mit Ammoniak oder primären oder sekundären Aminen der allgemeinen Formel III 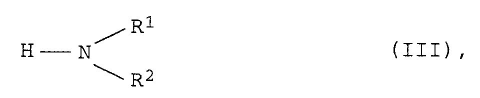 in der R¹ und R² die oben genannten Bedeutungen haben, bei Temperaturen von 200 bis 350°C und Drücken von 100 bis 300 bar in Gegenwart eines Heterogenkatalysators, dadurch gekennzeichnet, daß man als Heterogenkatalysator Zeolithe des Typs PSH-3, MCM-22, SSZ-25 oder deren Gemische einsetzt.

2. Verfahren zur Herstellung von Aminen I nach Anspruch 1, dadurch gekennzeichnet, daß man das gebildete Amin I abtrennt und die nichtumgesetzten Einsatzstoffe II und III zurückführt.

3. Verfahren zur Herstellung von Aminen nach den Ansprüchen 1 bis 2, dadurch gekennzeichnet, daß man als Olefin II Isobuten, Diisobuten, Cyclopenten, Cyclohexen oder Polyisobuten einsetzt.

4. Verfahren zur Herstellung von Aminen nach den Ansprüchen 1 bis 3, dadurch gekennzeichnet, daß man als Heterogenkatalysatoren MCM-22 Zeolithe in der H-Form einsetzt.

5. Verfahren zur Herstellung von Aminen nach den Ansprüchen 1 bis 4, dadurch gekennzeichnet, daß man als Heterogenkatalysatoren MCM-22 Zeolithe, die mit einer Säure, insbesondere einer aus der Gruppe Salzsäure, Flußsäure, Schwefelsäure, Phosphorsäure, Oxalsäure oder deren Gemischen behandelt sind, einsetzt.

6. Verfahren zur Herstellung von Aminen nach den Ansprüchen 1 bis 5, dadurch gekennzeichnet, daß man als Heterogenkatalysatoren MCM-22 Zeolithe, die mit einem oder mehreren Übergangsmetallen dotiert sind, einsetzt.

7. Verfahren zur Herstellung von Aminen nach den Ansprüchen 1 bis 6, dadurch gekennzeichnet, daß man als Heterogenkatalysatoren MCM-22 Zeolithe, die mit einem oder mehreren Elementen der Seltenen Erden dotiert sind, einsetzt.

8. Verfahren zur Herstellung von Aminen nach den Ansprüchen 1 bis 7, dadurch gekennzeichnet, daß man als Heterogenkatalysatoren MCM-22 Zeolithe in der Ammoniumform einsetzt.

9. Verfahren zur Herstellung von Aminen nach den Ansprüchen 1 bis 8, dadurch gekennzeichnet, daß man als Heterogenkatalysatoren MCM-22 Zeolithe einsetzt, die mit einem oder mehreren Elementen aus der Gruppe der Alkali-, Erdalkalimetalle oder Erdmetalle dotiert sind.

10. Verfahren zur Herstellung von Aminen nach den Ansprüchen 1 bis 9, dadurch gekennzeichnet, daß man als Heterogenkatalysatoren MCM-22 Zeolithe einsetzt, die mit einem Bindemittel verformt und bei Temperaturen von 200 bis 600°C calciniert sind.

11. Verfahren zur Herstellung von Aminen nach den Ansprüchen 1 bis 10, dadurch gekennzeichnet, daß man als Heterogenkatalysatoren dealuminierte MCM-22 Zeolithe einsetzt.

## Claims

1. A process for preparing amines of the general formula I 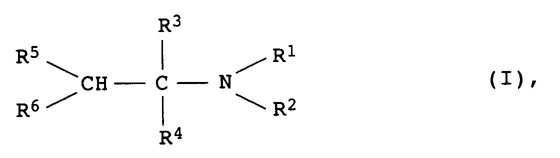 where
R¹,R²,R³,R⁴,R⁵ and R⁶ are each hydrogen, C₁-C₂₀-alkyl, C₂-C₁₂-alkenyl, C₂-C₈-alkynyl, C₃-C₁₂-cycloalkyl, C₄-C₁₂-alkylcycloalkyl, C₄-C₁₂-cycloalkylalkyl, aryl, C₇-C₁₆-alkylaryl or C₇-C₁₆-aralkyl,
R¹ and R² are together a saturated or unsaturated C₃-C₉-alkylene dichain, and
R³ and R⁵ are together a C₂-C₁₂-alkylene dichain,
by reacting olefins of the general formula II 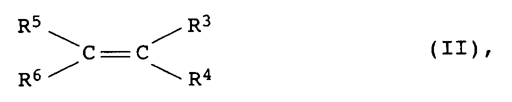 where R³, R⁴, R⁵ and R⁶ are each as defined above, with ammonia or primary or secondary amines of the general formula III 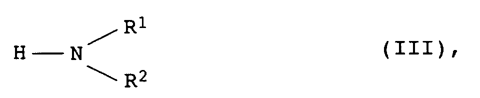 where R¹ and R² are each as defined above, at temperatures from 200 to 350°C and pressures from 100 to 300 bar in the presence of a heterogeneous catalyst, which comprises using a heterogeneous catalyst comprising zeolites of the type PSH-3, MCM-22 or SSZ-25 or mixtures thereof.

2. A process as claimed in claim 1, wherein the product amine I is separated off and the unconverted feed materials II and III are recycled.

3. A process as claimed in claim 1 or 2, wherein olefin II is isobutene, diisobutene, cyclopentene, cyclohexene or polyisobutene.

4. A process as claimed in any of claims 1 to 3, wherein the heterogeneous catalyst used is an MCM-22 zeolite in the H-form.

5. A process as claimed in any of claims 1 to 4, wherein the heterogeneous catalyst used is an MCM-22 zeolite which has been treated with an acid, in particular with an acid selected from the group consisting of hydrochloric acid, hydrofluoric acid, sulfuric acid, phosphoric acid, oxalic acid and mixtures thereof.

6. A process as claimed in any of claims 1 to 5, wherein the heterogeneous catalyst used is an MCM-22 zeolite doped with one or more transition metals.

7. A process as claimed in any of claims 1 to 6, wherein the heterogeneous catalyst used is an MCM-22 zeolite doped with one or more rare earth elements.

8. A process as claimed in any of claims 1 to 7, wherein the heterogeneous catalyst used is an MCM-22 zeolite in the ammonium form.

9. A process as claimed in any of claims 1 to 8, wherein the heterogeneous catalyst used is an MCM-22 zeolite doped with one or more elements from the group of the alkali, alkaline earth or earth metals.

10. A process as claimed in any of claims 1 to 9, wherein the heterogeneous catalyst used is an MCM-22 zeolite molded with a binder and calcined at from 200 to 600°C.

11. A process as claimed in any of claims 1 to 10, wherein the heterogeneous catalyst used is a dealuminated MCM-22 zeolite.

## Revendications

1. Procédé pour la préparation d'amines de formule générale I 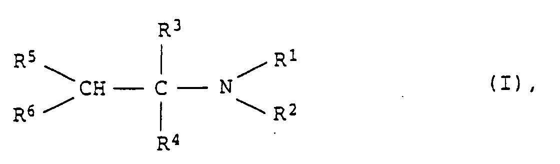 où
R¹, R², R³, R⁴, R⁵, R⁶ représentent l'hydrogène ou un groupe alkyle en C₁ à C₂₀, alcényle en C₂ à C₁₂, alcynyle en C₂ à C₈, cycloalkyle en C₃ à C₁₂, alkyl-cycloalkyle en C₄ à C₁₂, cycloalkyl-alkyle en C₄ à C₁₂, aryle, alkylaryle en C₇ à C₁₆ ou aralkyle en C₇ à C₁₆,
R¹ et R² représentent ensemble une chaîne divalente alkylène en C₃ à C₉, saturée ou insaturée, et
R³ et R⁵ représentent ensemble une chaîne divalente alkylène en C₂ à C₁₂,
par réaction d'oléfines de formule générale II 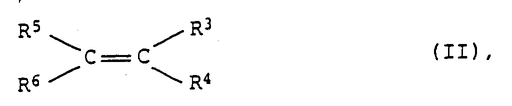 où R³, R⁴, R⁵ et R⁶ ont les significations indiquées ci-dessus, avec de l'ammoniac ou des amines primaires ou secondaires de formule générale III 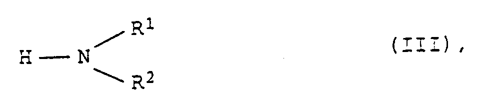 où R¹ et R² ont les significations indiquées ci-dessus, à des températures de 200 à 350°C et sous des pressions de 100 à 300 bar en présence d'un catalyseur hétérogène, caractérisé par le fait qu'on utilise comme catalyseur hétérogène des zéolites du type PSH-3, MCM-22, SSZ-25 ou leurs mélanges.

2. Procédé pour la préparation d'amines I selon la revendication 1, caractérisé par le fait qu'on sépare l'amine I forméè et on recycle les substances II et III n'ayant pas réagi.

3. Procédé pour la préparation d'amines selon les revendications 1 à 2, caractérisé par le fait qu'on utilise comme oléfines II de l'isobutène, du diisobutène, du cyclopentène, du cyclohexène ou du polyisobutène.

4. Procédé pour la préparation d'amines selon les revendications 1 à 3, caractérisé par le fait qu'on utilise comme catalyseurs hétérogènes des zéolites MCM-22 sous la forme H.

5. Procédé pour la préparation d'amines selon les revendications 1 à 4, caractérisé par le fait qu'on utilise comme catalyseurs hétérogènes des zéolites MCM-22, qui sont traitées avec un acide, en particulier avec un acide du groupe de l'acide chlorhydrique, de l'acide fluorhydrique, de l'acide sulfurique, de l'acide phosphorique, de l'acide oxalique ou de leurs mélanges.

6. Procédé pour la préparation d'amines selon les revendications 1 à 5, caractérisé par le fait qu'on utilise comme catalyseurs hétérogènes des zéolites MCM-22, qui sont dopées avec un ou plusieurs métaux de transition.

7. Procédé pour la préparation d'amines selon les revendications 1 à 6, caractérisé par le fait qu'on utilise comme catalyseurs hétérogènes des zéolites MCM-22, qui sont dopées avec un ou plusieurs éléments des terres rares.

8. Procédé pour la préparation d'amines selon les revendications 1 à 7, caractérisé par le fait qu'on utilise comme catalyseurs hétérogènes des zéolites MCM-22 sous forme ammonium.

9. Procédé pour la préparation d'amines selon les revendications 1 à 8, caractérisé par le fait qu'on utilise comme catalyseurs hétérogènes des zéolites MCM-22 qui sont dopées avec un ou plusieurs éléments du groupe des métaux alcalins, des métaux alcalino-terreux ou des terres rares.

10. Procédé pour la préparation d'amines selon les revendications 1 à 9, caractérisé par le fait qu'on utilise comme catalyseurs hétérogènes des zéolites MCM-22 qui sont traitées avec un liant et sont calcinées à des températures de 200 à 600°C.

11. Procédé pour la préparation d'amines selon les revendications 1 à 10, caractérisé par le fait qu'on utilise comme catalyseurs hétérogènes des zéolites MCM-22 désaluminées.
